# EUROPÄISCHE PATENTSCHRIFT

(11) **EP 1 374 756 B1**
(45) Veröffentlichungstag und Bekanntmachung des Hinweises auf die Patenterteilung: **23.08.2017**
(21) Anmeldenummer: 03013248.4
(22) Anmeldetag: 12.06.2003
(51) Int. Cl.: A61B 90/00, A61F 9/007

(54) **Kataraktchirurgie-Mikroskopiesystem und Verfahren hierzu**
Cataract surgery microscopy device and corresponding method
Dispositif de microscopie pour la chirurgie de la cataracte et méthode correspondante

(30) Priorität: 13.06.2002 DE 10226382
(43) Veröffentlichungstag der Anmeldung: 02.01.2004
(73) Patentinhaber: Carl Zeiss Meditec AG, 07745 Jena (DE)
(72) Erfinder: Pérez, Michel, Dr., F-21000 Dijon (FR); Kolster, Daniel, D-73447 Oberkochen (DE); Gaida, Gerhard, Dr., D-73430 Aalen (DE); Reimer, Peter, D-73479 Ellwangen (DE)
(74) Vertreter: Diehl & Partner GbR

(56) Entgegenhaltungen:
- EP-A- 0 882 438
- DE-U1- 9 301 448
- US-A- 4 490 022
- US-A- 4 964 715
- US-A- 5 054 907
- US-A- 5 801 807

## Beschreibung

Die Erfindung betrifft ein Kataraktchirurgie-Mikroskopiesystem und ein Verfahren zur Vorbereitung eines Eingriffs im Rahmen einer Kataraktoperation.
Bei einer Kataraktoperation wird eine natürliche Linse des menschlichen Auges, in welcher sich eine Katarakt entwickelt hat, durch eine künstliche Linse ersetzt. Dies ist ein mikrochirurgischer Eingriff, den ein Operateur unter Beobachtung mittels eines Operationsmikroskops vornimmt. Über einen Zugang durch die Sklera oder Cornea wird innerhalb des Innenrands der Iris und ohne Verletzung derselben eine Inzision in den Kapselsack eingebracht. Durch diese Inzision wird zum einen die körpereigene natürliche Linse beispielsweise nach Ultraschallzertrümmerung durch Absaugen entfernt und zum anderen die künstliche Linse eingesetzt.
Herkömmlicherweise wird die Inzision im Kapselsack nach freiem Augenmaß des Operateurs eingebracht, und zwar derart, daß die Inzision einen zickzackförmgigen oder in etwa kreisförmigen Verlauf um einen Mittelpunkt des Innenrands der Iris aufweist.
Es besteht nun die Vermutung, daß bestimmte Komplikationen als Spätfolgen der Katarktoperation dann mit geringerer Wahrscheinlichkeit auftreten, wenn die Inzision im Kapselsack eine im wesentlichen kreisförmige Gestalt aufweist, deren Durchmesser an den Durchmesser der einzusetzenden künstlichen Linse angepaßt ist.

US 5,801,807 offenbart eine Beleuchtungsvorrichtung für ophthalmologische Zwecke, in deren Strahlengang eine variable Blende angeordnet ist, um Bereiche eines untersuchten Auges vor exzessivem Lichteinfall zu schützen.

US 5,054,907 offenbart eine ophthalmologische Diagnosevorrichtung, bei der vordefinierte Muster auf die Cornea projiziert werden und von der Cornea reflektierte Muster hinsichtlich ihrer Deformation analysiert werden, um eine Gestalt bzw. Topologie der Cornea zu ermitteln.

Es ist eine Aufgabe der vorliegenden Erfindung, ein Kataraktchirurgie-Mikroskopiesystem, ein Verfahren zur Vorbereitung eines Eingriffs im Rahmen einer Kataraktoperation und ein Verfahren zum Durchführen einer Kataraktoperation vorzuschlagen, welche es dem Operateur erleichtern, diese Vorgabe zu erfüllen.

Unter einem ersten Aspekt der Erfindung ist ein Kataraktchirurgie-Mikroskopiesystem gemäß den beiliegenden Ansprüchen 1 oder 2 bereitgestellt. Vorteilhafte Ausführungsformen sind in den abhängigen Ansprüchen 3 bis 8 definiert. Unter einem zweiten Aspekt der Erfindung ist ein Verfahren zum Vorbereiten einer Kataraktoperation gemäß beiliegendem Anspruch 9 bereitgestellt. Vorteilhafte Ausführungsformen hierzu sind in den abhängigen Ansprüchen 10 bis 12 definiert.

Ein Operateur kann das Mikroskopiesystem bei der Durchführung der Inzision in dem Kapselsack einsetzen und dabei das Mikroskopiesystem derart relativ zu dem operierten Auge ausrichten, daß der Kapselsack und die Iris in etwa in der Objektebene der Mikroskopieoptik angeordnet sind, so daß er deren Bild durch die Mikroskopieoptik betrachten kann. Er kann das Mikroskopiesystem derart einstellen, daß das generierte Ringmuster in dem Bild innerhalb des Innenrands der Iris sichtbar wird und die Inzision in dem Kapselsack entlang dem Ringmuster vornehmen. Das Ringmuster ist somit eine Hilfe, um eine Inzision mit einer gewünschten Gestalt zu erzeugen.

Das Ringmuster kann insbesondere ein Kreismuster sein, welches einen einzigen, von der Orientierung unabhängigen Durchmesser aufweist. Es kann jedoch auch ein ovales Muster sein, welches nach Art einer Ellipse einen maximalen und einen minimalen Durchmesser aufweist. Das Ringmuster kann jedoch auch ein aus Polygonstücken zusammengesetztes Muster sein. Hierbei kommt es darauf an, daß der bildliche Eindruck, den das Ringmuster beim Operateur hervorruft, geeignet ist, für den Operateur eine Hilfestellung zur Führung des Schneidwerkzeugs bei der Durchführung der Inzision ist.

Die Durchmesser des Ringmusters werden dabei derart eingestellt, daß die Gestalt der entsprechend durchgeführten Inzision auf eine Gestalt einer in den Kapselsack einzusetzenden Linse abgestimmt ist.

Vorzugsweise entspricht einer der Durchmesser, vorzugsweise der maximale Durchmesser, der Inzision in etwa dem 0,5fachen bis 0,6fachen, dem 0,6fachen bis 0,7fachen, dem 0,7fachen bis 0,8fachen, dem 0,8fachen bis 0,9fachen oder dem 0,9fachen bis 1,0fachen Durchmesser der einzusetzenden Linse.

Zur Erleichterung einer Ausrichtung des Ringmusters in dem Bild relativ zu dem Kapselsack ist der Mustergenerator vorzugsweise derart ausgebildet, daß er ferner ein Referenzmuster generiert, welches eine Mehrzahl von Teilmustern aufweist, welche mit einem jeweils gleichen einstellbaren Abstand von einem Zentrum des Referenzmusters angeordnet sind. Der Operateur kann den Abstand in etwa derart einstellen, daß er in dem Bild in etwa dem Radius des Innenrands der Iris entspricht, und er kann dann weiter die Mikroskopieoptik relativ zu dem Auge derart ausrichten, daß die Teilmuster jeweils nahe dem Innenrand der Iris angeordnet sind oder mit diesem im wesentlichen überlagert sind.

Insbesondere ist dann weiter vorgesehen, daß das Zentrum des Referenzmusters mit einem Mittelpunkt des Ringmusters zusammenfällt, so daß auch die entlang dem Kreismuster vorgenommene Inzision zentrisch bezüglich der Iris erzeugt wird.

Der Mustergenerator weist Schnittstellen zur Eingabe der Durchmesser des Ringmusters und zur Eingabe des Abstands der Teilmuster des Referenzmusters von dessen Zentrum auf. Da der Durchmesser der Inzision an den Durchmesser der einzusetzenden Linse angepaßt ist, liegt der wenigstens eine Durchmesser des Ringmusters meist bereits vor dem Eingriff fest und kann beispielsweise über eine Tastatur in das System eingegeben werden.

Der Innendurchmesser der Iris des Auges ist meist nicht vordefiniert und hängt von verschiedenen Parametern, wie etwa einer Medikamentierung des Patienten, ab. Entsprechend ist es vorteilhaft, für die Einstellung des Abstands der Teilmuster des Referenzmusters von dessen Zentrum eine Schnittstelle vorzusehen, welche von einer den Eingriff vorbereitenden Person einfach bedienbar ist. Vorzugsweise umfaßt die Schnittstelle zur Einstellung des Abstands ein Stellglied, welches an einem Gehäusekörper der Mikroskopieoptik angeordnet ist.

Unter einem weiteren Aspekt sieht die Erfindung ein Verfahren zur Vorbereitung eines Eingriffs im Rahmen einer Kataraktoperation vor, welches ein Erzeugen eines mikroskopischen Bildes derart umfaßt, daß wenigstens ein Innenrand einer Iris eines zu operierenden Auges in dem Bild sichtbar ist, und welches weiter ein Einblenden eines Ringmusters in das Bild derart umfaßt, daß das Ringmuster einen Verlauf einer in einer Haut eines Kapselsacks des Auges einzubringenden Inzision repräsentiert.

Ausführungsformen der Erfindung werden nachfolgend anhand von Zeichnungen näher erläutert. Hierbei zeigt
- Figur 1: eine schematische Darstellung einer Ausführungsform eines erfindungsgemäßen KataraktchirurgieMikroskopiesystems,
- Figur 2: eine Darstellung eines von dem Mikroskopiesystem der Figur 1 erzeugten Bildes,
- Figur 3: eine Variante eines in Figur 2 gezeigten Ringmusters,
- Figur 4: eine weitere Variante des in Figur 2 gezeigten Ringmusters, ein vergleichendes Beispiel für ein Kataraktchirurgie.
- Figur 5: ein vergleichendes Beispiel für ein Kataraktchirurgie-Mikroskopiesystem, und
- Figur 6: eine Variante eines Details des in Figur 5 gezeigten Systems.

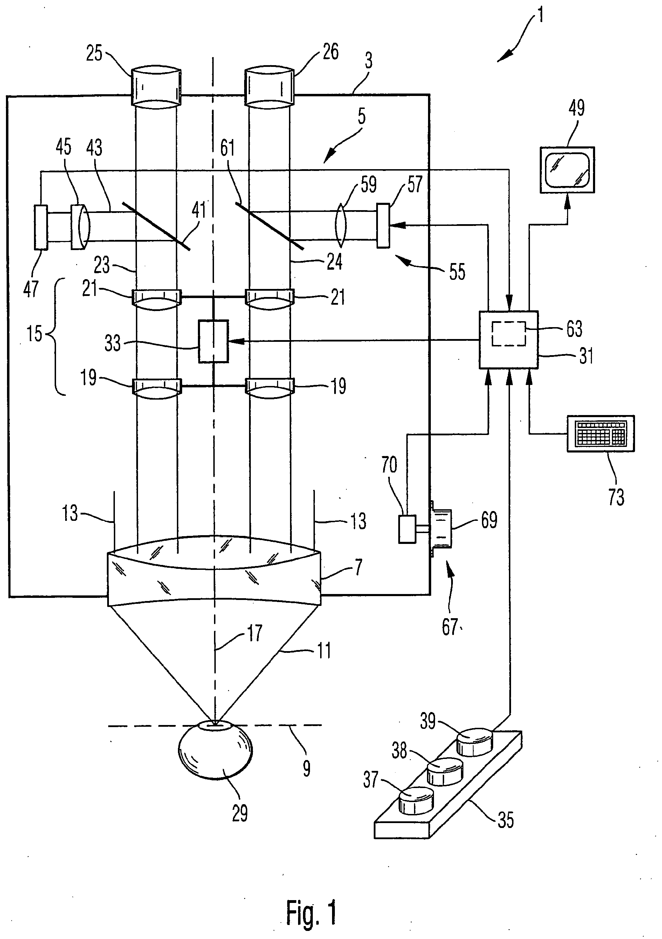

In Figur 1 ist ein Kataraktchirurgie-Mikroskopiesystem 1 schematisch dargestellt. Dieses umfaßt einen Gehäusekörper 3, in dem eine Mikroskopieoptik 5 aufgenommen ist. Die Mikroskopieoptik 5 umfaßt ein Mikroskopieobjektiv 7, welches ein von einer Objektebene 9 des Objektivs 7 ausgehendes objektseitiges divergentes Strahlenbündel 11 in ein bildseitiges paralleles Strahlenbündel 13 überführt. Aus diesem greift ein doppelt ausgelegtes Zoomsystem 15 mit entlang einer optischen Achse 17 des Objektivs 7 verlagerbaren Linsengruppe 19 und 21 zwei Teilstrahlenbündel 23 und 24 heraus, welche Okularen 25 bzw. 26 zugeführt werden, in welche der Operateur mit seinem linken und rechten Auge einblicken kann, um ein Bild der Objektebene 9 zu betrachten.

Zur Durchführung einer Kataraktoperation ordnet der Operateur das Mikroskopiesystem 1 vor einem Auge 29 eines Patienten, an dem die Operation durchzuführen ist, an. Der Operateur schafft zunächst einen Zugang zu einem Kapselsack des Auges, beispielsweise dadurch, daß entsprechende Inzisionen an einer Sklera oder Cornea des Auges 29 durchgeführt werden. Sodann wird die Inzision an dem Kapselsack vorbereitet. Hierzu umfaßt das Mikroskopiesystem 1 eine Steuerung 31, beispielsweise einen Personalcomputer, welcher einen Motor 33 zur Verlagerung der Linsengruppen 19 und 21 des Zoomsystem 15 und damit zur Änderung einer Vergrößerung der Mikroskopieoptik 5 ansteuert. Kommandos zur Ansteuerung des Motors 33 erhält die Steuerung 31 von einem Schalterpult 35 mit Drucktastern 37, 38 und 39, welche vom Fuß des Operateurs oder der Person, die die Inzision an dem Kapselsack vorbereitet, betätigbar sind. Wird der Drucktaster 37 gedrückt, steuert die Steuerung 31 den Motor 33 derart an, daß die Vergrößerung der Mikroskopieoptik 35 erhöht wird. Bei Drücken des Drucktasters 38 wird die Vergrößerung der Mikroskopieoptik entsprechend erniedrigt.

Das Mikroskopiesystem 1 umfaßt ferner einen halbdurchlässigen Spiegel 41, welcher in dem Teilstrahlenbündel 23 angeordnet ist, um aus diesem ein Strahlenbündel 43 auszukoppeln, welches über eine Adapteroptik 45 auf einen Kamerachip 47 derart geleitet wird, daß auf diesem ein Bild der Objektebene 9 entsteht. Von dem Kamerachip 47 aufgenommene Bilder werden von der Steuerung 31 ausgelesen und auf einem Bildschirm 49 dargestellt. Damit ist auf dem Bildschirm 49 das gleiche Bild der Objektebene 9 sichtbar, wie es ein Betrachter beim Einblick in das Okular 25 wahrnimmt.

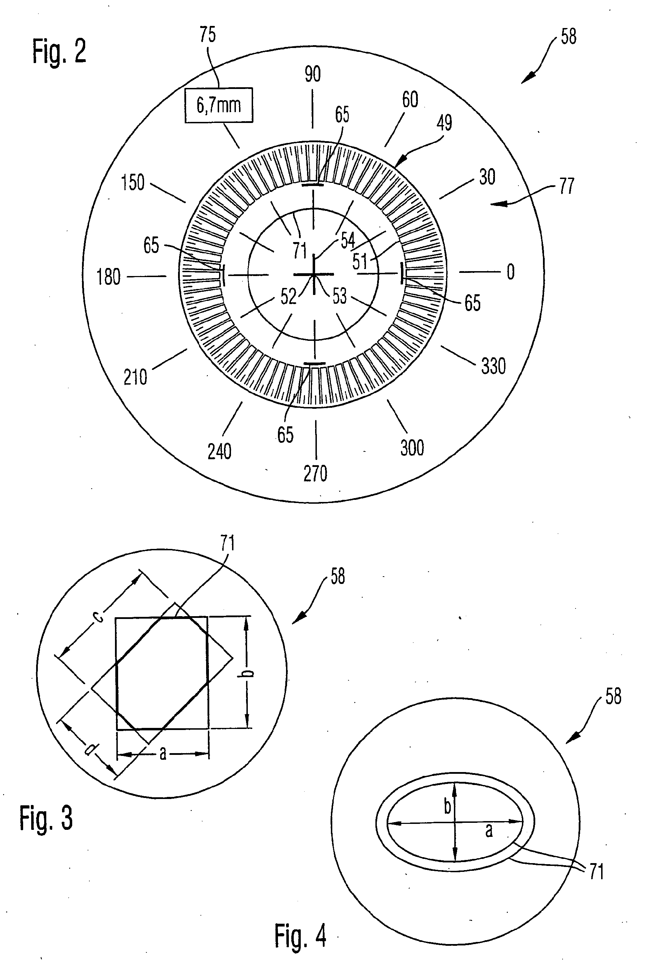

Die die Inzision vorbereitende Person kann dann durch Betätigen der Drucktaster 37 und 38 die Vergrößerung derart ändern und die Mikroskopieoptik 5 relativ zu dem Auge 29 derart ausrichten, daß das Operationsfeld in angemessener Größe von der Mikroskopieoptik 5 abgebildet wird bzw. auf dem Bildschirm 49 dargestellt ist.
Eine schematische Darstellung eines Beispiels eines hierbei erzeugten Bildes 58 ist in Figur 2 dargestellt. Darin bezeichnet die Bezugsziffer 49 eine Iris des Auges 29 mit einem Innenrand 51 und einem Zentrum 53.

Das Mikroskopiesytem 1 umfaßt ferner einen Projektor 55 mit einer Anzeigevorrichtung 57, beispielsweise einer LCD-Anzeige, eine Projektionsoptik 59 und einem halbdurchlässigen Spiegel 61. Der halbdurchlässige Spiegel 61 ist in dem Teilstrahlenbündel 24 angeordnet und koppelt ein durch die Anzeigevorrichtung 57 dargestelltes und von der Optik 59 projiziertes Muster in das Teilstrahlenbündel 24 derart ein, daß es beim Einblick in das Okular 26 in überlagerter Darstellung mit dem Bild der Objektebene 9 wahrgenommen wird. Das von der Anzeigevorrichtung 57 dargestellte Muster wird durch einen Mustergenerator 63 in der Steuerung erzeugt, wobei die Steuerung 31 dieses Muster auch dem Bild überlagert, welches auf dem Bildschirm 49 dargestellt wird. Das von dem Mustergenerator 63 erzeugte Muster umfaßt ein Referenzmuster, welches aus vier Teilmustern 65 zusammengesetzt ist. Die Teilmuster 65 sind in dem Bild der Figur 2 mit gleichem Abstand von einem Mittelpunkt 52 angeordnet und bilden mit gleichem Abstand in Umfangsrichtung angeordnete Teile eines Kreises um diesen Mittelpunkt 52. Der Abstand zwischen den Teilmustern 65 und dem Zentrum 53 ist einstellbar und wird von der Steuerung 31 über eine Schnittstelle 67 eingelesen, welche einen an dem Gehäusekörper 3 angebrachten Drehknopf 69 und einen Drehstellungsgeber 70 umfaßt, dessen Stellungssignal von der Steuerung 31 ausgelesen wird. Die den Eingriff vorbereitende Person stellt durch Betätigung des Drehknopfs 69 den Abstand der Teilmuster 65 von dem Mittelpunkt 52 derart ein, daß die Teilmuster 65 nahe und symmetrisch zu dem Innenrand 51 der Iris 49 des Auges 29 angeordnet sind. Bei dieser Einstellung ist dann auch die Mikroskopieoptik 5 relativ zu dem Auge 29 derart angeordnet, daß der Mittelpunkt 52 des Referenzmusters mit seinen Teilmustern 65 mit dem Zentrum 53 der Iris 49 zusammenfällt, das heißt die Mikroskopieoptik 5 ist bezüglich der Iris 49 zentriert.

Der Mustergenerator 63 erzeugt weiter ein Ringmuster, welches in der vorliegend beschriebenen Ausführungsform die Gestalt eines Kreismusters 69 aufweist, dessen Mittelpunkt ebenfalls mit dem Zentrum 53 zusammenfällt. Ein Radius des Kreismusters 69 beträgt 6,7 mm und wurde von der den Eingriff vorbereitenden Person über eine Tastatur 73 in die Steuerung 31 eingegeben. Der Wert von 6,7 mm entspricht dem 0,8fachen des Durchmessers der in den Kapselsack einzusetzenden künstlichen Linse und wird in das Bild auch als numerische Darstellung in einem Feld 75 von dem Mustergenerator 63 dargestellt.

Der Mustergenerator 63 erzeugt ferner noch ein Grad-Bogenmuster 77, aus welchem Winkelpositionen in Umfangsrichtung um das Zentrum 53 ablesbar sind.

Ein Betriebsmodus des Mustergenerators 63 ist durch Betätigen des Drucktasters 39 einstellbar. In einem ersten Betriebsmodus erzeugt der Mustergenerator 63 lediglich das Kreismuster 71 und keine weiteren Muster, das heißt er erzeugt das Referenzmuster 65 und das Grad-Bogenschema 77 nicht. In einem zweiten Betriebsmodus erzeugt der Mustergenerator 63 lediglich das Kreismuster 71 und ein Kreuz 54 im Zentrum 53. In einem dritten Betriebsmodus erzeugt der Mustergenerator 63 lediglich das Kreismsuter 71 und das Referenzmuster mit den Teilmustern 65. In einem vierten Betriebsmuster erzeugt der Mustergenerator 63 lediglich das Kreismuster 71, das Referenzmuster mit Teilmustern 65 und das Kreuzmuster 54. In einem fünften Betriebsmodus erzeugt der Mustergenerator 63 sämtliche vorangehend beschriebene Muster, nämlich das Kreismuster 71, das Referenzmuster mit Teilmustern 65, das Kreuzmuster 54 und das Grad-Bogenschema 77. In einem sechsten Betriebsmodus erzeugt der Mustergenerator 63 keines der in dem fünften Betriebsmodus dargestellten Muster und der Operateur erhält somit ein Bild des Operationsfelds, welches frei von jeglichen in den Strahlengang eingekoppelten Mustern ist. Mit einer jeden Betätigung des Drucktasters 39 wird der Mustergenerator 63 sequentiell von einem Betriebsmodus in den nächsten geschaltet.

Hierbei werden die Muster 65 und 71 von dem Mustergenerator 63 derart erzeugt, daß deren Größen mit der von dem Motor 33 eingestellten Vergrößerung der Mikroskopieoptik skalieren. Damit wird auch bei Änderungen der Vergrößerung der Mikroskopieoptik 5 eine relative Größe der Iris 49 und der Muster 65 bzw. 71 beibehalten. Hierbei ist es möglich, das Gradbogenmuster 77 ebenfalls mit der Vergrößerung zu skalieren, oder aber das Gradbogenmuster 77 nicht zu skalieren und unabhängig von der eingestellten Vergrößerung in gleichbleibender Größe darzustellen.

Nach der vorangehend beschriebenen Vorbereitung des Eingriffs ist in dem Okular 26 bzw. auf dem Bildschirm 49 ein Bild sichtbar, wie es schematisch in Figur 2 wiedergegeben ist.

Der Operateur kann dann die Inzision in dem Kapselsack vornehmen, indem er in die Okulare 25 und 26 Einblick nimmt und die Inzision entlang dem ihm in dem Okular 26 dargestellten Kreismuster 71 durchführt. Nachfolgend wird er dann durch die entstandene Öffnung in dem Kapselsack die bestehende natürliche Linse, beispielsweise durch Ultraschall zertrümmern und absaugen, entfernen und sodann die künstliche intraokulare Linse in den Kapselsack durch die entstandene Öffnung einfügen. Durch die Hilfestellung durch das in dem Bild dargestellte Kreismuster 71 ist es dem Operateur gelungen, eine an den Durchmesser der künstlichen Linse angepaßte Inzision vorzunehmen, so daß die Wahrscheinlichkeit für spätere Komplikationen reduziert ist.

In der vorangehend beschriebenen Ausführungsform ist das dem Bild überlagerte Ringmuster ein Kreismuster. Es ist jedoch auch möglich, dem Operateur als Hilfe zur Führung der Inzision beispielsweise ein Polygonmuster darzubieten. Ein Beispiel hierfür ist in Figur 3 schematisch dargestellt, wo sich das Polygon als Achteck durch die Überlagerung zweier Rechtecke ergibt. Die Rechtecke haben unterschiedliche Kantenlängen bzw. Durchmesser a, b und c, d.

Eine weitere Variante für ein Ringmuster ist in Figur 4 schematisch dargestellt, wo das Ringmuster aus zwei mit einem kleinen radialen Abstand voneinander angeordneten Ellipsen besteht. Der Operateur kann dann die Inzision z.B. in den Zwischenraum zwischen den beiden Ellipsen legen.

Es ist auch möglich, daß der Mustergenerator das Ringmuster nicht ständig als Ganzes darstellt, sondern zeitlich nacheinander einzelne Teile des Ringmusters darstellt. Insbesondere ist es möglich, daß der Mustergenerator einen "Leuchtpunkt" erzeugt, der sich entlang der Kontur des Ringmusters bewegt und diese in einem Zeitraum von beispielsweise 5 Sekunden abtastet.

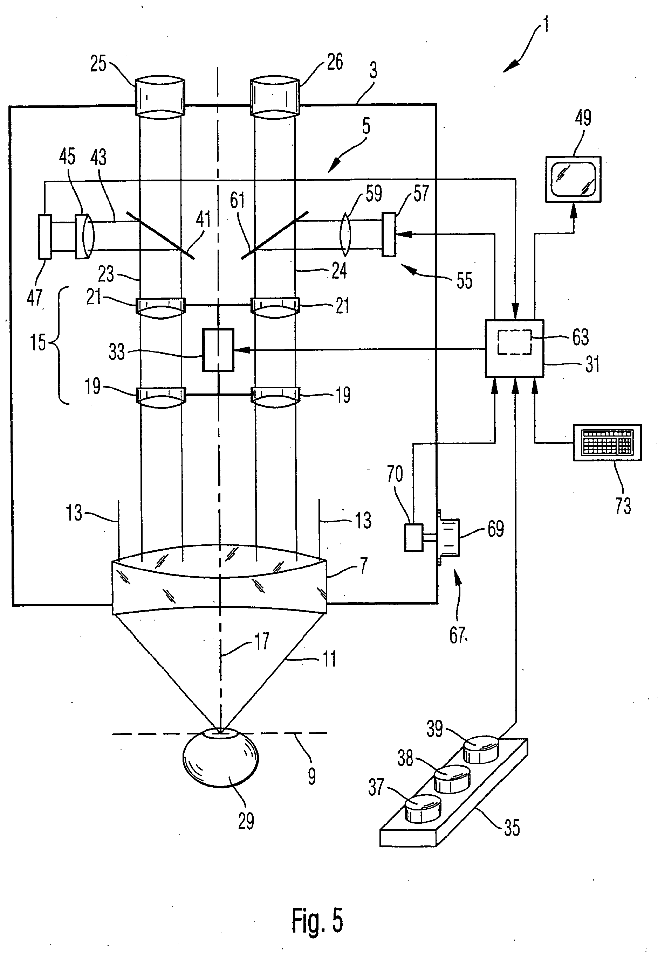

Figur 5 zeigt eine weitere Variante der anhand der Figur 1 erläuterten Ausführungsform. Das in Figur 5 schematisch Figur 5 zeigt ein vergleichendes Beispiel für ein Kataraktchirurgie-Mikroskopiesystem. Das in Figur 5 schematisch dargestellte Kataraktchirurgie-Mikroskopiesystem 1 ähnelt weitgehend dem in Figur 1 gezeigten System. Es unterscheidet sich von diesem jedoch dadurch, daß der Mustergenerator das Muster 71 nicht hin zum Okular 26 projiziert, sondern entgegengesetzt in das Teil-strahlenbündel 24 derart einkoppelt, daß in der Objektebene 9 ein Bild des Musters entsteht. Damit wird das Muster 71 auf einen Bereich um den zu operierenden Kapselsack projiziert, und aufgrund einer Rückstreuung dieses Bereichs erkennt der Operateur bei Betrachtung des Kapselsacks durch das Mikroskopiesystem dort einen hell leuchtenden Bereich, der das Muster 71 wiedergibt. Er kann sich auch dann an dem Muster 71 orientieren, um die Inzision am Kapselsack durchzuführen.

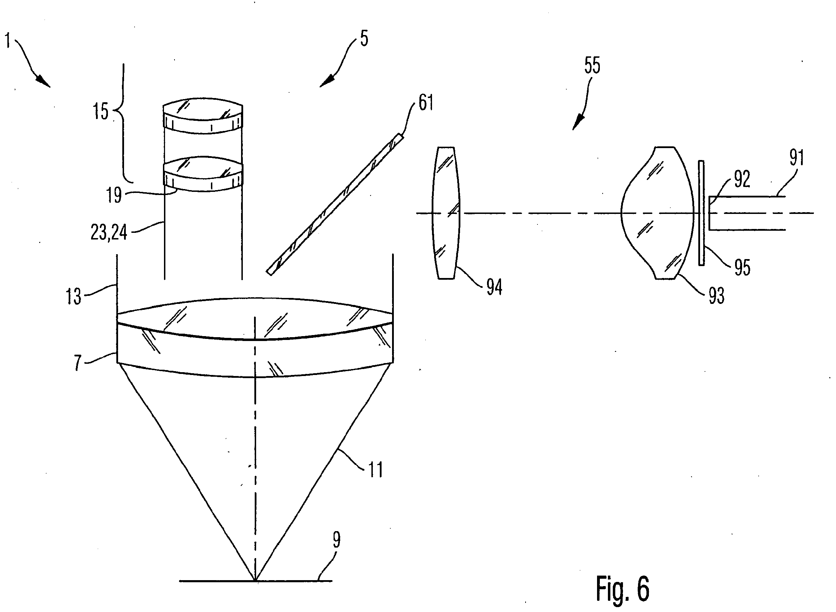

In Figur 6 ist eine Detailansicht einer Variante der anhand der Figur 5 erläuterten Beispiels schematisch dargestellt. Hierbei wird das Ringmuster in eine Mikroskopieoptik 5 eines Kataraktchirurgie-Mikroskopie-systems 1 nicht über eine der Teilstrahlenbündel 23, 24, welche zu in Figur 6 nicht dargestellten Okularen des Mikroskopiesystems 1 führen, eingekoppelt, sondern unabhängig von diesen Teilstrahlenbündeln 23, 24 direkt in einen Strahlengang eines bildseitigen Strahlenbündels 13. So umfaßt ein Projektionssystem 55 des in Figur 6 gezeigten Mikroskopiesystems 1 ein Lichtleiter-Faserbündel 91, mit welchem üblicherweise Licht zur Beleuchtung einer Objektebene 9 der Mikroskopieoptik 5 zugeführt wird. Das Licht tritt aus einem Ende 92 des Lichtleiter-Faserbündels 91 aus und wird durch asphärische Linse 93 sowie eine weitere Linse 94 kollimiert und auf einen Umlenkspiegel 61 gerichtet, welcher das kollimierte Licht in das Strahlenbündel 13 einkoppelt, allerdings in Richtung hin zu dem Objektiv 7. Das Licht durchsetzt das Objektiv 7 und wird zu der Objektebene 9 hingeleitet.

Zwischen dem Ende 92 des Lichtleiter-Faserbündels 91 und der asphärischen Linse 93 ist ein LCD-Display 95 angeordnet, welches von einem in Figur 6 nicht dargestellten Rechner angesteuert wird. Die Optik aus der asphärischen Linse 93, der Linse 94 und dem Objektiv 7 ist derart eingestellt, daß ein auf dem LCD-Display 95 dargestelltes Muster von dem aus dem Lichtleiter-Faser-bündel 91 austretenden Licht auf die Objektebene 9 abge-bildet wird. Somit entsteht in der Objektebene 9 ein Abbild des auf dem LCD-Display 95 dargestellten Musters. Wenn der Rechner mit einem darin enthaltenen Mustergenerator das im Zusammenhang mit den vorangehenden beschriebenen Aus-führungsformen erläuterte Ringmuster erzeugt, so wird dieses in die Objektebene 9 abgebildet. Ist dort das zu operierende Auge mit seinem Kapselsack angeordnet, so entstehen, da der Kapselsack streuende Strukturen aufweist, Rückreflexe, welche vom Operateur beim Einblick in das Mikroskopiesystem 1 wahrgenommen werden können und welche dem Operateur eine Hilfestellung bei der durchzuführenden Inzision bieten.

Es ist auch möglich, das LCD-Display 95 an einer anderen als der in Figur 6 gezeigten Stelle im Strahlengang des Beleuchtungsstrahls anzuordnen, beispielsweise zwischen den Linsen 93 und 94 oder zwischen der Linse 94 und dem Spiegel 61.

Bei dem vorangehend beschriebenen Verfahren und der Mikroskopieoptik ist das Zentrum 53 des Innenrands 51 der Iris 49 bzw. das Zentrum 52 des Referenzmusters mit seinen Teilmsutern 65 bezüglich dem Mittelpunkt des Kreismusters 71 zentriert, um die Inzision zentriert bezüglich der Iris vorzunehmen. Es ist jedoch auch möglich, den Mittelpunkt des Kreismusters 71 etwas außerhalb des Zentrums 52 der Teilmuster 65 bzw. des Innenrands 51 der Iris 49 anzuordnen.

Vorangehend wurde das Ringmuster 71 durch eine durchgehende Linie dargestellt. Es ist jedoch auch möglich, für die Darstellung des Ringmusters 71 eine unterbrochene Linie, wie etwa eine gestrichelte Linie oder strichpunktierte Linie, zu verwenden.

Ferner wurde vorangehend das Referenzmuster durch lediglich vier Teilmuster 65 dargestellt. Es ist jedoch auch möglich, eine größere Zahl von Teilmustern einzusetzen, welche mit jeweils gleichem Abstand von dem Mittelpunkt des Referenzmusters angeordnet sind. Insbesondere ist es möglich, das Referenzmuster durch eine zusammenhängende Kreislinie darzustellen.

In der vorangehend beschriebenen Ausführungsform wird der Durchmesser des Referenzmusters mit den Teilmustern 65 über die Tastatur 73 eingestellt. Es ist jedoch auch möglich, für die Einstellung dieses Durchmessers ein Stellglied am Hauptkörper 3 der Mikroskopieoptik vorzusehen, ähnlich wie dies für die Einstellung der Durchmesser des Ringmusters 71 beschrieben wurde. Andererseits ist es auch möglich, den Durchmesser des Ringmusters 71 über die Tastatur 73 einzugeben.

Neben den vorangehend geschilderten manuellen Einstellungen für Lage des Zentrums des Referenzmusters und dessen Durchmessers oder/und der Lage des Mittelpunkts des Ringmusters bzw. dessen Durchmessers kann auch ein automatisches Verfahren eingesetzt werden, welches mit Hilfe einer Bild-verarbeitung den Innenrand der Iris erkennt und die Einstellungen automatisch vornimmt.

In der vorangehend beschriebenen Ausführungsform ist der halbdurchlässige Spiegel 41 zur Auskopplung des Strahlenbündels 43 und zur Erzeugung eines Bildes der Objektebene 9 durch den Kamerachip 47 in dem Teilstrahlenbündel 23 angeordnet, während der halbdurchlässige Spiegel 61 zur Einkopplung des durch die Anzeigevorrichtung 57 dargestellten Musters in den Strahlengang der Mikroskopieoptik 5 in dem Teilstrahlenbündel 24 angeordnet ist. Es ist jedoch auch möglich, die beiden halbdurchlässigen Spiegel 41 und 61 in lediglich einem der Teilstrahlenbündel 23 oder 24 vorzusehen.
Ebenso ist es möglich, in beide Teilstrahlenbündel 23, 24 das Ringmuster insbesondere derart einzublenden, daß beim Betrachter ein stereomikroskopischer Eindruck des Ringmusters in der Objektebene oder gegebenenfalls etwas darüber oder etwas darunter entsteht.
Bei der vorangehend beschriebenen Ausführungsform führt der Operateur den Eingriff während des Einblicks in die beiden Okulare 25 und 26 der Mikroskopieoptik 5 durch. Es ist jedoch auch möglich, daß der Operateur den Eingriff während der Betrachtung des Operationsfeldes, das heißt der Objektebene 9, auf dem Bildschirm 49 vornimmt.
Ferner ist möglich, daß die Steuerung 31 das dem Bildschirm 49 zugeführte Bild auch einer Anzeige zuführt, welche fest an dem Kopf des Operateurs angebracht ist und welche als "head mounted display" bezeichnet wird. Dann erhält der Operateur auf ergonomische Weise ein Bild des Operationsfeldes und der von dem Mustergenerator 63 erzeugten Muster, ohne mit seiner Kopfstellung durch die Anordnung der Mikroskopieoptik 5 festgelegt zu sein.

## Patentansprüche

1. Kataraktchirurgie-Mikroskopiesystem, umfassend:
eine Mikroskopieoptik (5) mit einem Mikroskopobjektiv (7) und einem Okular (26) zum Erzeugen eines Bilds (58) einer Objektebene (9) der Mikroskopieoptik (5), wobei ein halbdurchlässiger Spiegel (61) in einem Strahlengang zwischen dem Mikroskopobjektiv (7) und dem Okular (26) angeordnet ist, und
einen Mustergenerator (63) zur Erzeugung eines dem Bild (58) überlagerten Musters (71, 65, 77, 54),
wobei der Mustergenerator (63) derart ausgebildet ist, daß er ein Ringmuster (71) mit wenigstens einem einstellbaren Durchmesser generiert, wobei der Mustergenerator eine Anzeigevorrichtung (57) und eine Projektionsoptik (59) umfaßt, um das Ringmuster über den halbdurchlässigen Spiegel (61) zu projzieren,
**dadurch gekennzeichnet,**
**dass** der halbdurchlässige Spiegel (61) dazu konfiguriert ist, das Ringmuster (71) hin zu dem Okular (26) zu projizieren.

2. Kataraktchirurgie-Mikroskopiesystem, umfassend:
eine Mikroskopieoptik (5) zum Erzeugen eines Bilds (58) einer Objektebene (9) der Mikroskopieoptik (5), und
einen Mustergenerator (63) zur Erzeugung eines Musters (71, 65, 77, 54),
wobei der Mustergenerator (63) derart ausgebildet ist, daß er ein Ringmuster (71) mit wenigstens einem einstellbaren Durchmesser generiert,
**dadurch gekennzeichnet,**
**dass** das Kataraktchirurgie-Mikroskopiesystem (1) einen Bildschirm (49) umfasst, und
**dass** die Mikroskopieoptik (5) dazu konfiguriert ist, das Bild (58) der Objektebene (9) auf dem Bildschirm (49) zu erzeugen und das Ringmuster (71) auf dem Bildschirm (49) in Überlagerung mit dem Bild der Objektebene (9) darzustellen.

3. Kataraktchirurgie-Mikroskopiesystem nach Anspruch 1 oder 2, wobei der Mustergenerator (63) eine Schnittstelle (73) zur Eingabe des wenigsten einen Durchmessers des Ringmusters (71) umfaßt.

4. Kataraktchirurgie-Mikroskopiesystem nach Anspruch 3, wobei die Mikroskopieoptik eine Mikroskopieoptik (5) mit einstellbarer Vergrößerung ist und der Mustergenerator (63) mit der Mikroskopieoptik (5) gekoppelt und derart ausgebildet ist, daß der Durchmesser des Ringmusters (71) mit der Vergrößerung in Abhängigkeit von der eingestellten Vergrößerung änderbar ist, insbesondere proportional zu der eingestellten Vergrößerung einstellbar ist.

5. Kataraktchirurgie-Mikroskopiesystem nach einem der Ansprüche 1 bis 4, wobei der Mustergenerator (63) derart ausgebildet ist, daß er ein Referenzmuster mit einer Mehrzahl von Teilmustern (65) generiert, welche jeweils mit einem gleichen einstellbaren Abstand von einem Zentrum (52) des Referenzmusters angeordnet sind.

6. Kataraktchirurgie-Mikroskopiesystem nach Anspruch 5, wobei das Zentrum (52) des Referenzmusters fest bezüglich einem Mittelpunkt des Ringmusters angeordnet ist und insbesondere mit dem Mittelpunkt zusammen-fällt.

7. Kataraktchirurgie-Mikroskopiesystem nach Anspruch 6 oder 5, wobei der Mustergenerator eine Schnittstelle (67) zur Eingabe des Abstands umfaßt.

8. Kataraktchirurgie-Mikroskopiesystem nach Anspruch 7, wobei die Schnittstelle (67) zur Eingabe des Abstands einen von einem Benutzer betätigbares Stellglied (69) an einem Gehäusekörper (3) der Mikroskopieoptik (5) aufweist.

9. Verfahren zur Vorbereitung eines Eingriffs im Rahmen einer Kataraktoperation, umfassend:
Erzeugen eines mikroskopischen Bildes (58) eines zu operierenden Auges (29) derart, daß wenigstens ein Innenrand (51) einer Iris (49) des Auges (29) in dem Bild (58) sichtbar ist, und
Einblenden eines Ringmusters (71) in das Bild (58).

10. Verfahren nach Anspruch 9, wobei ein Durchmesser des Ringmusters (71) derart eingestellt wird, daß er einem 0,5-fachen bis 1,0-fachen eines Durchmessers einer in einen Kapselsack des Auges (29) einzusetzenden Linse oder einem Radialfortsatz einer solchen Linse entspricht.

11. Verfahren nach Anspruch 10, ferner umfassend:
Einblenden eines Referenzmusters mit einer Mehrzahl von Teilmustern (65) in das Bild (58), wobei die Teilmuster (65) jeweils mit einem gleichen einstellbaren Abstand von einem Zentrum (52) des Referenzmusters angeordnet sind.

12. Verfahren nach Anspruch 11, wobei der Abstand derart eingestellt wird und die Mikroskopieoptik relativ zu dem Auge derart ausgerichtet wird, daß die Teilmuster (65) in dem Bild (58) nahe dem Innenrand (51) der Iris (49) angeordnet sind oder mit diesem im wesentlichen überlagert sind.

## Claims

1. Cataract surgery microscopy system, comprising:
a microscopy optical unit (5) with a microscope objective (7) and an eyepiece (26) for producing an image (58) of an object plane (9) of the microscopy optical unit (5), wherein a semi-transparent mirror (61) is arranged in a beam path between the microscope objective (7) and the eyepiece (26), and a pattern generator (63) for producing a pattern (71, 65, 77, 54) that is superimposed on the image (58), wherein the pattern generator (63) is embodied in such a way that it generates a ring pattern (71) with at least one adjustable diameter, wherein the pattern generator comprises a display apparatus (57) and a projection optical unit (59) for projecting the ring pattern via the semi-transparent mirror (61), **characterized in that** the semi-transparent mirror (61) is configured to project the ring pattern (71) towards the eyepiece (26).

2. Cataract surgery microscopy system, comprising:
a microscopy optical unit (5) for producing an image (58) of an object plane (9) of the microscopy optical unit (5) and a pattern generator (63) for producing a pattern (71, 65, 77, 54), wherein the pattern generator (63) is embodied in such a way that it generates a ring pattern (71) with at least one adjustable diameter, **characterized**
**in that** the cataract surgery microscopy system (1) comprises a screen (49) and in that the microscopy optical unit (5) is configured to produce the image (58) of the object plane (9) on the screen (49) and to present the ring pattern (71) on the screen (49) in a manner superimposed on the image of the object plane (9).

3. Cataract surgery microscopy system according to Claim 1 or 2, wherein the pattern generator (63) comprises an interface (73) for entering the at least one diameter of the ring pattern (71).

4. Cataract surgery microscopy system according to Claim 3, wherein the microscopy optical unit is a microscopy optical unit (5) with an adjustable magnification and the pattern generator (63) is coupled to the microscopy optical unit (5) and embodied in such a way that the diameter of the ring pattern (71) is changeable with the magnification in a manner dependent on the set magnification, in particular said diameter of the ring pattern can be set in a manner proportional to the set magnification.

5. Cataract surgery microscopy system according to any one of Claims 1 to 4, wherein the pattern generator (63) is embodied in such a way that it generates a reference pattern with a plurality of partial patterns (65), which are respectively arranged with a same adjustable distance from a centre (52) of the reference pattern.

6. Cataract surgery microscopy system according to Claim 5, wherein the centre (52) of the reference pattern is fixedly arranged in relation to a centre point of the ring pattern and, in particular, coincides with the centre point.

7. Cataract surgery microscopy system according to Claim 6 or 5, wherein the pattern generator comprises an interface (67) for entering the distance.

8. Cataract surgery microscopy system according to Claim 7, wherein the interface (67) for entering the distance has an actuator (69) that is actuatable by a user, said actuator being situated on a housing body (3) of the microscopy optical unit (5).

9. Method for preparing an intervention within the scope of a cataract operation, comprising the following steps:
producing a microscopic image (58) of an eye (29) to be operated on, in such a way that at least one inner edge (51) of an iris (49) of the eye (29) is visible in the image (58), and overlaying a ring pattern (71) onto the image (58).

10. Method according to Claim 9, wherein a diameter of the ring pattern (71) is set in such a way that it corresponds to 0.5-times to 1.0-times a diameter of a lens that is to be inserted into a capsular bag of the eye (29) or a radial projection of such a lens.

11. Method according to Claim 10, furthermore comprising the following step:
overlaying a reference pattern with a plurality of partial patterns (65) onto the image (58), wherein the partial patterns (65) are each arranged at the same adjustable distance from a centre (52) of the reference pattern.

12. Method according to Claim 11, wherein the distance is set and the microscopy optical unit is aligned relative to the eye in such a way that the partial patterns (65) in the image (58) are arranged in the vicinity of the inner edge (51) of the iris (49) or are substantially overlaid thereon.

## Revendications

1. Système de microscopie pour la chirurgie de la cataracte, comprenant :
une optique de microscopie (5) comportant un objectif de microscopie (7) et un oculaire (26) destinés à générer une image (58) d'un plan objet (9) de l'optique de microscopie (5), dans lequel un miroir semi-transparent (61) est disposé sur un chemin de faisceau entre l'objectif de microscopie (7) et l'oculaire (26), et
un générateur de motif (63) destiné à générer un motif (71, 65, 77, 54) superposé à l'image (58),
dans lequel le générateur de motif (63) est réalisé de manière à générer un motif annulaire (71) ayant au moins un diamètre réglable, dans lequel le générateur de motif comprend un dispositif d'affichage (57) et une optique de projection (59) destinée à projeter le motif annulaire par l'intermédiaire du miroir semi-transparent (61),
**caractérisé en ce que** le miroir semi-transparent (61) est configuré de manière à projeter le motif annulaire (71) vers à l'oculaire (26).

2. Système de microscopie pour la chirurgie de la cataracte, comprenant :
une optique de microscopie (5) destinée à générer une image (58) d'un plan objet (9) de l'optique de microscopie (5), et
un générateur de motif (63) destiné à générer un motif (71, 65, 77, 54),
dans lequel le générateur de motif (63) est réalisé de manière à ce qu'il génère un motif annulaire (71) ayant au moins un diamètre pouvant être ajusté,
**caractérisé en ce que** le système de microscopie pour la chirurgie de la cataracte (1) comprend un écran (49), et **en ce que**
l'optique de microscopie (5) est configurée de manière à générer l'image (58) du plan objet (9) sur l'écran (49) et à représenter le motif annulaire (71) sur l'écran (49) de manière superposée à l'image du plan objet (9).

3. Système de microscopie pour la chirurgie de la cataracte selon la revendication 1 ou 2, dans lequel le générateur de motif (63) comprend une interface (73) destinée à fournir en entrée l'au moins un diamètre du motif annulaire (71).

4. Système de microscopie pour la chirurgie de la cataracte selon la revendication 3, dans lequel l'optique de microscopie est une optique de microscopie (5) ayant un grossissement pouvant être ajusté et le générateur de motif (63) est couplé à l'optique de microscopie (5) et est réalisé de manière à ce que le diamètre du motif annulaire (71) puisse être modifié par l'intermédiaire du grossissement en fonction du grossissement ajusté, notamment de manière à ce qu'il puisse être ajusté proportionnellement au grossissement ajusté.

5. Système de microscopie pour la chirurgie de la cataracte selon l'une quelconque des revendications 1 à 4, dans lequel le générateur de motif (63) est réalisé de manière à ce qu'il génère un motif de référence comportant une pluralité de motifs partiels (65) qui sont respectivement disposés à une même distance pouvant être ajustée d'un centre (52) du motif de référence.

6. Système de microscopie pour la chirurgie de la cataracte selon la revendication 5, dans lequel le centre (52) du motif de référence est disposé en position fixe par rapport à un centre du motif annulaire et coïncide notamment avec ledit centre.

7. Système de microscopie pour la chirurgie de la cataracte selon la revendication 6 ou 5, dans lequel le générateur de motif comprend une interface (67) destinée à fournir en entrée la distance.

8. Système de microscopie pour la chirurgie de la cataracte selon la revendication 7, dans lequel l'interface (67) comporte un actionneur (69) pouvant être actionné destiné à fournir en entrée la distance d'un utilisateur par rapport à un corps de boîtier (3) de l'optique de microscopie (5).

9. Procédé de préparation d'une intervention dans le cadre d'une opération de la cataracte, consistant à :
générer une image microscopique (58) d'un oeil à opérer (29) de manière à ce qu'au moins un bord intérieur (51) d'un iris (49) de l'oeil (29) soit visible dans l'image (58), et
fusionner un motif annulaire (71) dans l'image (58).

10. Procédé selon la revendication 9, dans lequel un diamètre du motif annulaire (71) est ajusté de manière à ce qu'il corresponde à 0,5 fois à 1,0 fois un diamètre d'une lentille devant être mise en place dans un sac capsulaire de l'oeil (29) ou à un prolongement radial d'une telle lentille.

11. Procédé selon la revendication 10, consistant en outre à:
fusionner un motif de référence comportant une pluralité de motifs partiels (65) dans l'image (58), dans lequel les motifs partiels (65) sont respectivement disposés à une même distance pouvant être ajustée d'un centre (52) du motif de référence.

12. Procédé selon la revendication 11, dans lequel la distance est réglée, et l'optique de microscopie est orientée par rapport à l'oeil, de manière à ce que les motifs partiels (65) soient disposés dans l'image (58) à proximité du bord intérieur (51) de l'iris (49) ou à ce qu'ils soient sensiblement superposés à celui-ci.
